# EUROPEAN PATENT APPLICATION

(11) **EP 1 419 795 A1**
(43) Date of publication of application: **19.05.2004**
(21) Application number: 03025124.3
(22) Date of filing: 03.11.2003
(51) Int. Cl.: A61M 16/22, A62B 19/00

(54) **Absorber arrangement for anesthesia apparatus**

(30) Priority: 13.11.2002 SE 0203342
(71) Applicant: Maquet Critical Care AB, 171 95 Solna (SE)
(72) Inventor: Videbrink, Petter, 194 34 Upplands Väsby (SE)
(74) Representative: Samzelius, Roger Mikael

(57) **Abstract**

An absorber arrangement (6) for an anesthesia apparatus, which absorber arrangement (6) comprises a docking unit (10) and a cassette unit (8), removably connected to the docking unit (10), wherein the docking unit (10) includes a valve (14) adapted to shut off flow paths (12) in the absorber arrangement (6) when the cassette unit (8) is detached from the docking unit (10) is described. Increased functionality with fewer components is made possible by the valve (14) being formed by a shunt valve adapted to by-pass the flow paths (12) in the absorber arrangement (6) in dependence of the position of the cassette unit (8) in the docking unit (10) and the cassette unit (8) being freely positionable between a first position and a second position, whereby the by-pass is 100% for the cassette unit (8) in the first position and is 0% for the cassette unit (8) in the second position.

## Description

The present invention relates to an absorber arrangement according to the preamble of Claim 1.

Anesthesia apparatus may be designed with many different types of breathing circuits that in principle can be categorised as more or less open and closed. At the one extreme there is the completely open system in which all of the breathing gas that comes from a patient during exhalation is evacuated. This results in a large consumption of expensive anesthetic and is therefore seldom used.

At the other extreme there is the completely closed system. In this system carbon dioxide is removed from the exhaled breathing gas, which is then re-supplied to the patient with the addition of gases to compensate for those taken up by the patient's body via the lungs, primarily oxygen. The closed system uses substantially smaller quantities of the expensive anesthetic but requires a greater degree of control and monitoring.

The majority of "closed" systems therefore utilise greater gas consumption than is necessary, in order to make the control and monitoring easier.

The removal of carbon dioxide is done in absorber arrangements connected to the flow paths of the breathing circuits, for example such as described in WO 92/05826. The absorber arrangement includes a carbon dioxide absorber that, when full, must be exchanged. The absorber arrangement therefore normally comprises a docking unit and a cassette unit, the latter being exchangeable. In order to prevent the escape of breathing gas from the breathing circuit as the exchange of the cassette section takes place a valve for shutting off flow paths within the absorber arrangement can be arranged in the docking section. This results in all of the exhaled gas being re-supplied to the patient during the time that it takes for the cassette unit to be exchanged. Advantageously, the valve may be designed so that it automatically shuts the flow paths when the cassette unit is disconnected.

However there exist situations in which a certain re-breathing of carbon dioxide containing gas is desirable. Supplying carbon dioxide after the absorber arrangement could, of course, solve this but an even simpler solution is to by pass couple a portion of the exhaled breathing gas past the absorber arrangement. Such a solution is described in SE 506 727 where a shunt valve is coupled in parallel with the absorber arrangement so as to be able to by-pass couple an adjustable amount of gas past the absorber arrangement. However, the system that is described in SE 506 727 lacks the possibility of shutting off the flow paths to the absorber arrangement when the cassette unit is changed.

An aim of the present invention is to provide an absorber arrangement by which a change of the cassette unit may be simply and safely achieved without the risk of leakage and also by which an adjustable amount of the exhaled breathing gas may be coupled past the absorber arrangement in a simple manner.

This aim is achieved in accordance with the present invention by means of the above absorber arrangement being devised according to the characterising portion of Claim 1.

Advantageous refinements and embodiments are set out in the claims dependent on Claim 1.

By designing the shut off valve in the docking unit so that it may be also employed as a shunt valve, regulated by the position of the cassette unit, a simple and a robust system is achieved that with a minimum of components carries out many of the functions that the previously known systems employed separate components for.

An absorber arrangement according to the invention shall be described in more detail and with reference to the accompanying figures, of which:
FIG. 1 shows an absorber arrangement according to the invention; and
FIG. 2 shows a valve of the absorber arrangement.

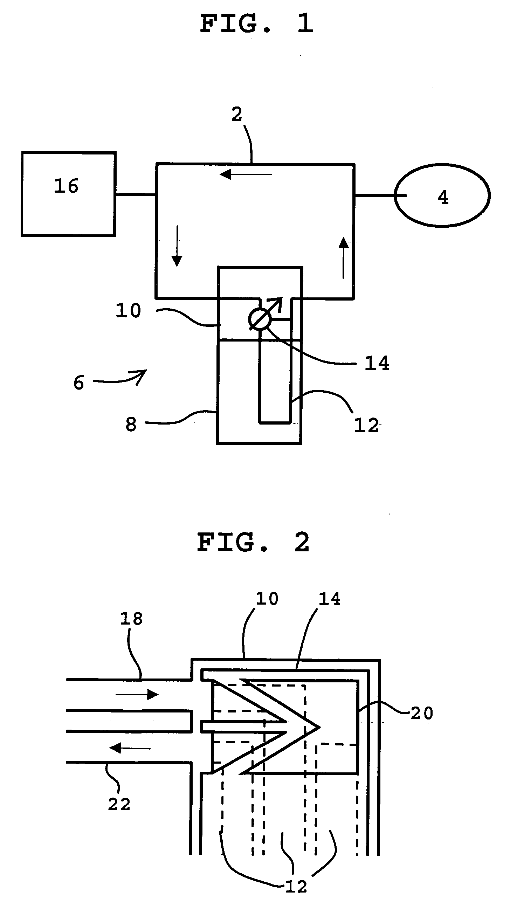

A breathing circuit 2 in an anesthetic apparatus is shown in FIG. 1. The breathing circuit 2 is connected to a patient 4. Arrows indicate the flow direction for breathing gas within the breathing circuit 2.

An absorber arrangement 6 is arranged in the breathing circuit 2 to absorb carbon dioxide from the breathing gas before it is led to the patient 4. The absorber arrangement 6 comprises a cassette unit 8 and a docking unit 10. The cassette unit 8 can be disconnected from the docking unit 10 when the absorber (not shown) in the cassette unit 8 is saturated with carbon dioxide. In the present embodiment the cassette unit 8 is disconnected through a rotational movement of the cassette unit 8 about its own axis.

Flow paths 12 in the absorber arrangement 6 have been indicated in the figure purely schematically. A valve 14 is arranged in the flow paths 12 between the docking unit 10 and the cassette unit 8. The valve 14 fulfils two primary functions.

The valve 14 works in part as an automatic shut off valve when the cassette unit 8 is disconnected from the docking unit 10. All of the gas then flows directly to the breathing circuit 2.

The valve 14 works in part as a shunt valve. When the cassette unit 8 is connected to the docking unit 10 the valve 14 operates to gradually connect in the flow paths 12 until the cassette unit 8 has reached its end position (in this case rotation-wise). By locating the cassette unit 8 between these two positions a selectable portion of the breathing gas flow may be by-pass coupled past the flow paths 12 in the cassette unit 8.

FIG. 2 shows the valve 14 in greater detail. The docking unit 10 is indicated whilst the cassette unit is omitted. The breathing gas is provided to the absorber arrangement via an inlet 18 and on to the valve 14. A shunt element 20 in the valve 14 regulates how much of the flow shall pass through the flow paths 12 and how much shall pass directly to an outlet 22. Adjustment is made dependent on the position (rotational position) of the shunt element 20. In one extreme position all of the gas is provided to the flow paths 12 and in the other extreme position all of the gas is provided directly to the outlet 22 (in this latter position the cassette unit can be disengaged from the docking unit 10).

As is shown in FIG. 2 the flow paths 12 are co-axially arranged, which eases the construction when a rotation of the shunt element 20 is employed to regulate the flow.

The same effect of the double functionality of closing and by-pass coupling may be achieved using other types of valves based on other directional movements, for example linear movement.

## Claims

1. An absorber arrangement (6) for an anesthesia apparatus, which absorber arrangement (6) comprises a docking unit (10) and a cassette unit (8), removably connected to the docking unit (10), wherein the docking unit (10) includes a valve (14) adapted to shut off flow paths (12) in the absorber arrangement (6) when the cassette unit (8) is detached from the docking unit (10), **characterised in that** the valve (14) is formed by a shunt valve adapted to by-pass the flow paths (12) in the absorber arrangement (6) in dependence of the position of the cassette unit (8) in the docking unit (10) and **in that** the cassette unit (8) is freely positionable between a first position and a second position, whereby the by-pass is 100% for the cassette unit (8) in the first position and is 0% for the cassette unit (8) in the second position.

2. An absorber arrangement according to Claim 1, **characterised in that** the valve (14) is adapted for rotation and **in that** the cassette unit (8) is connectable to the valve (14).

3. An absorber arrangement according to Claim 1 or Claim 2 **characterised in that** the valve (14) is formed with co-axially arranged flow paths (12).
